# EUROPEAN PATENT APPLICATION

(11) **EP 4 817 620 A1**
(43) Date of publication of application: **07.10.2026**
(21) Application number: 23962899.3
(22) Date of filing: 29.12.2023
(51) Int. Cl.: A61K 35/12, C12N 5/078, A61P 25/28, A61P 39/00, A61P 3/00

(54) **MITOCHONDRIAL TRANSPLANTATION THERAPY SYSTEM AND USE THEREOF**

(71) Applicant: Guangzhou Institutes of Biomedicine and Health, Chinese Academy of Sciences, Guangzhou, Guangdong 510530 (CN); GUANGZHOU MEDICAL UNIVERSITY, Guangzhou, Guangdong 511436 (CN)
(72) Inventor: LIU, Xingguo, Guangzhou, Guangdong 510530 (CN); DU, Shiwei, Guangzhou, Guangdong 510530 (CN); LONG, Qi, Guangzhou, Guangdong 510530 (CN); ZHOU, Yanshuang, Guangzhou, Guangdong 510530 (CN)
(74) Representative: Studio Torta S.p.A.
(86) International application number: PCT/CN2023/143578
(87) International publication number: WO 2025/138225

(57) **Abstract**

Belonging to the technical field of biomedical materials, the present disclosure provides a mitochondrial transplantation therapy system and its applications. The system is characterized by comprising isolated mitochondria and a transfer vector. The transfer vector includes at least one of an artificial lipid vesicle, a cell-derived vesicle, or an exosome. The system is formed by assembling these components. The mitochondrial transplantation therapy system has a simple preparation process and can efficiently and quickly transfer healthy mitochondria into cells or tissues simultaneously, repairing mitochondrial functions of mitochondrial-damaged cells. The mitochondrial transplantation therapy system has potential to treat a disease associated with mitochondrial dysfunction.

## Description

### FIELD

The present disclosure relates to the technical field of biomedical materials, specifically to a mitochondrial transplantation therapy system and its use.

### BACKGROUND

Mitochondria are semi-autonomously replicating organelles that supply energy in eukaryotic cells, and play a vital role in ATP production, cellular metabolism, growth and development, as well as aging and death. Human mitochondrion harbors a circular doublestranded DNA of about 16.5 kb, known as mitochondrionl DNA (mtDNA), which encode 13 proteins, 2 rRNAs, and 22 tRNAs. These proteins are core subunits of mitochondrial respiratory complexes I, III to V, and are crucial for maintaining normal cellular activities. The oxidative phosphorylation in mitochondria exposes mtDNA to a highly reactive oxygen species(ROS) environment. Meanwhile, mtDNA lacks the protection of histones. Therefore, compared with nuclear DNA, mtDNA is highly susceptible to oxidative damage, leading to mitochondrial dysfunction. Gene mutations can also occur during the replication of mitochondrial DNA. While these mutations are normally eliminated by quality control mechanisms like mitophagy and mitochondrial fusion-fission dynamics, they accumulate within cells when mitochondria become dysfunctional. This accumulation further increases the overall mutational burden. Mitochondrial DNA exhibits a critical threshold effect. Once the mutation rate of mtDNA reaches the threshold, mitochondrial functions are severely impaired, consequently triggering cell death or tissue lesions, which can ultimately manifest as mitochondrial disease. Mitochondrial dysfunction, especially dysfunction in metabolic activities, is associated with a wide range of pathologies, including metabolic disease, neurodegenerative disease, and cancer, as well as an aging process.

Mitochondrial disease is a genetic disorder that is largely inherited through mtDNA mutations, with a prevalence of approximately 1 in 5,000. A hallmark of these diseases is heteroplasmy-the coexistence of mutant and wild-type mtDNA within cells. Disease severity is directly correlated with the mtDNA mutation load due to a threshold effect. A distinct characteristic is that symptoms typically requires that more than 60% of mtDNA carries mutations, underscoring this critical threshold. Currently, numerous researchers are attempting to develop therapeutic strategies aim to reduce the mtDNA mutation rate below the pathogenic threshold, offering hope for treating these largely incurable diseases. The clinical landscape of mitochondrial disorders is notably complex, characterized by heterogeneity and tissue specificity, where identical mutations can result in vastly different phenotypes. Some medicaments have been used to alleviate the symptoms of the mitochondrial disease. For example, idebenone is used to treat Leber's hereditary optic neuropathy (LHON), while sodium valproate can alleviate symptoms of myoclonus epilepsy associated with mitochondrial dysfunction. Beyond symptomatic treatment, mitochondrial replacement therapy (MRT) offers a preventive approach by enabling individuals with inherited mitochondrial disease to have genetically related healthy children. Beyond existing treatments, several innovative therapeutic strategies are under investigation, including novel small-molecule medicament and gene therapy designed to directly target the mitochondrial genome. A particularly advanced approach is mitochondrial transplantation technology, which involves transplanting healthy mitochondria into damaged cells or organs to restore cellular energy metabolism and cure the disease. Currently, this strategy has shown positive outcomes in preclinical studies for a range of conditions, such as the neurodegenerative disease, Alzheimer's disease, Parkinson's disease, sarcopenia, and heart transplantation.

However, current mitochondrial transplantation technology faces several significant limitations. For instance, inappropriate selection of vectors can lead to low payload capacity (carrying an insufficient number of healthy mitochondria), poor delivery efficiency, or even damage to the structure or function of mitochondria. Therefore, there is a pressing need to refine and optimize the mitochondrial transplantation technology.

### SUMMARY

The present disclosure aims to solve at least one of the technical problems in the related art in the field of mitochondrial therapy. Accordingly, it is an objective of the present disclosure is to provide a new mitochondrial transplantation system and its applications. The system provided in the present disclosure has a straightforward preparation process and enables the highly efficient, rapid, and simultaneous delivery of healthy mitochondria into target cells or tissues, thereby repairing the mitochondrial function of the mitochondrial-damaged cells. The mitochondrial transplantation therapy system has potential to treat a disease rooted in mitochondrial dysfunction.

A first aspect of the present disclosure provides a mitochondrial transplantation therapy system. According to embodiments of the present disclosure, the system includes: isolated mitochondria; and a transfer vector. The mitochondrial transplantation therapy system is obtained by assembling the mitochondria with the transfer vector. The transfer vector is selected from the group consisting an artificial lipid vesicle, a cell-derived vesicle, or an exosome, and any combination thereof.

Cell membrane is a semipermeable barrier that defines the boundary of the cell. Its fundamental structure is a phospholipid bilayer, which provides a scaffold for various proteins, lipids, and carbohydrates. This structure exhibits excellent fluidity, enabling dynamic processes such as the formation of vesicles for transporting materials across the membrane, thereby ensuring a stable environment for cellular activities. Currently, due to its excellent biocompatibility and degradability, the cell membranes have promising applications in the field of drug development. Red blood cells (RBCs), the predominant cell type in blood, are essential for cellular respiration as they facilitate the transport of oxygen and carbon dioxide. A key feature of RBCs is that during maturation, the nucleus and organelles gradually disappear.

Parkinson's disease (PD) is a neurodegenerative disorder primarily affecting middle-aged and elderly people, and is associated with the degeneration of dopaminergic neurons in the substantia nigra. The neurotoxic substance 1-methyl-4-phenyl-1,2,3,6-tetrahydropyridine (MPTP) is converted to its active metabolite, MPP⁺ in brain. MPP⁺ is then selectively taken up by dopaminergic neurons in the substantia nigra, inhibiting the activity of mitochondrial respiratory chain complex I, which triggers mitochondrial oxidative stress, leading to dopaminergic neurons death and ultimately the manifestation of Parkinson's symptoms. Therefore, mitochondrial dysfunction is considered a likely contributor to Parkinson's disease. To investigate the therapeutic potential of mitochondrial transplantation *in vivo*, we established a PD mouse model using MPTP. The results showed that the mitochondrial transplantation therapy system provided in the present disclosure can restore motor function and neuronal function in Parkinson's disease mice.

At present, researchers have developed various methods to deliver mitochondria into cells. The simplest approach involves direct co-incubation of isolated mitochondria with recipient cells, allowing uptake via cellular endocytosis. However, this method has relatively low efficiency in delivering the mitochondria into the cells, and the internalized mitochondria often assume a spherical morphology, and fail to regain their native reticular network structure. The natural phenomenon of mitochondrial transfer between cells provides a rationale for therapeutic transplantation. Current delivery techniques, including microinjection, FluidFM technology, and photothermal nanoblades have been developed to transfer isolated mitochondria into target cells. However, these methods are constrained by single-cell operational complexity, low scalability, and stringent technical requirements, limiting their therapeutic utility. The present disclosure employs artificial lipid vesicle, cell-derived vesicle, or exosome as transfer vector. The developed mitochondrial transplantation therapy system is operationally simple and enables the efficient, rapid and simultaneous transfer mitochondria into a large number of target cells, thereby achieving the purpose of mitochondrial transfer.

According to the embodiments of the present disclosure, the transfer vector is a vesicular structure derived from red blood cells, selected from a native red blood cell membrane vesicle or a reconstituted lipid vesicle comprising red blood cell membrane components.

According to embodiments of the present disclosure, the mitochondrial transplantation therapy system is prepared by assembling extracted mitochondria with lipid vesicle derived from red blood cell membrane. The inventors found in experiments that when isolated mitochondria were co-incubated with other recipient cells, only a minimal degree of spontaneous mitochondrial transfer occurs. To overcome this inherent limitation and achieve high-efficiency delivery, the present system employs engineered red blood cell membrane vesicles as functional transfer vectors. Moreover, time-lapse imaging showed that the mitochondria were spherical and immotile in recipient cells, with no observable improvement in cellular viability or function. In particular, mitochondria encapsulated within the specific transfer vector of the present disclosure can efficiently enter the cells, and regain dynamic function, and confer a measurable functional benefit to the cells.

According to the embodiments of the present disclosure, the mitochondria are isolated from a mammalian cell.

A second aspect of the present disclosure provides a medicament. According to embodiments of the present disclosure, the medicament includes the mitochondrial transplantation therapy system described in the first aspect.

A third aspect of the present disclosure provides use of the mitochondrial transplantation therapy system described in the first aspect, or the medicament according to the second aspect in the manufacture of a medicament for repairing mitochondrial damage.

The inventors have found that upon co-incubation with mitochondrial-damaged cells, the mitochondrial transplantation therapy system in the present disclosure enables the efficient and rapid transfer of functional mitochondria into range of recipient cells. The transplanted mitochondria successfully integrate into the endogenous mitochondrial network, restore its structure, and, restore the network structure, and resume mitochondrial functions, thereby repairing mitochondrial damage of the recipient cells.

According to the embodiments of the present disclosure, the mitochondrial damage includes the mitochondrial dysfunction and an mtDNA mutation.

According to the embodiments of the present disclosure, the mitochondrial functional damage includes mitochondrial structural damage and mitochondrial metabolic damage.

According to the embodiments of the present disclosure, the mtDNA mutation includes an mtDNA point mutation and an mtDNA deletion mutation.

According to the embodiments of the present disclosure, the mitochondrial transplantation therapy system provided in the present disclosure can repair mtDNA damage mutations. Specifically, the inventors have found that the function of transplanted mitochondria was detected in mtDNA-depleted cells (ρ0 cells) and in GM04516 cells with large-fragment mtDNA deletions. Following mitochondrial transplantation, both cell lines exhibited comprehensive functional recovery. The mitochondrial morphology, mtDNA quantity, and membrane potential of the ρ0 cells were restored, alongside a significantly recovery of ATP production, and a transcription level of mtDNA-encoded proteins. Similarly, in GM04516 cells, transplantation restored the mitochondrial morphology, significantly increased mitochondrial ATP production, and rescued the transcription level of mtDNA-encoded proteins.

A fourth aspect of the present disclosure provides the use of mitochondrial transplantation therapy system according to the first aspect, or the medicament according to the second aspect, in the manufacture of a medicament for treating a disease associated with mitochondrial dysfunction.

The inventors have demonstrated that the mitochondrial transplantation therapy system described herein can repair the disease associated with mitochondrial dysfunction, such as Parkinson's disease. To assess the system's efficacy, the inventors constructed a Parkinson's disease mouse model by inducing mitochondrial damage with the small-molecule medicament MPTP. Subsequently, it was found that the mitochondrial transplantation therapy system could repair this damage. The results demonstrated marked restoration of both dopaminergic neurons and motor function in the treated mice, indicating the system's efficacy in repairing mitochondrial damage.

According to the embodiments of the present disclosure, the mitochondrial dysfunction is caused by impairment of mitochondrial respiratory chain, aberrations in nuclear-encoded proteins, or alterations in mitochondrial physicochemical properties.

According to the embodiments of the present disclosure, the disease associated with the mitochondrial dysfunction includes at least one of a nervous system disease, hearing impairment-related disease, optic neuropathy, myopathy, cardiac dysfunction, liver dysfunction, kidney dysfunction, pancreatic dysfunction, gastrointestinal dysfunction, metabolic disease, reproductive disease, or skeletal lesion attributable to the mitochondrial dysfunction.

According to the embodiments of the present disclosure, nervous system diseases include, but are not limited to, neuronal development delay, Parkinson's disease, Alzheimer's disease, stroke, epilepsy, migraine, and amyotrophic lateral sclerosis.

According to the embodiments of the present disclosure, the hearing impairment-related disease includes at least one of deafness or sensorineural hearing loss.

According to the embodiments of the present disclosure, the optic neuropathy includes at least one of progressive external ophthalmoplegia, optic atrophy, or retinitis pigmentosa.

According to the embodiments of the present disclosure, the myopathy includes at least one of progressive muscle weakness and atrophy, sarcopenia, or exercise intolerance. Optionally, the cardiac dysfunction includes at least one of cardiomyopathy or a myocardial conduction defect.

According to the embodiments of the present disclosure, the liver dysfunction includes at least one of liver failure, liver damage, fatty liver, or liver cirrhosis.

According to the embodiments of the present disclosure, the liver dysfunction includes at least one of Fanconi syndrome, renal tubular acidosis, glomerulosclerosis, renal failure, or adrenal insufficiency.

According to the embodiments of the present disclosure, the pancreatic dysfunction includes at least one of diabetes mellitus or pancreatitis.

According to the embodiments of the present disclosure, the gastrointestinal dysfunction includes at least one of intestinal pseudo-obstruction or gastrointestinal motility disorder.

According to the embodiments of the present disclosure, the metabolic disease includes at least one of senility, obesity, hyperglycemia, dyslipidemia, insulin resistance, or cardiovascular disease.

According to the embodiments of the present disclosure, the reproductive disease includes at least one of premature ovarian failure or male infertility.

According to the embodiments of the present disclosure, the skeletal disease includes at least one of kyphoscoliosis, short stature, or bone marrow failure.

A fourth aspect of the present disclosure provides use of the mitochondrial transplantation therapy system described in the first aspect, or the medicament according to the second aspect, in the manufacture of a medicament for: (1) skin repair; (2) alleviating ischemia-reperfusion injury associated with organ transplantation; (3) prolonging a preservation duration of a transplanted organ; and (4) treating or alleviating organ metabolic dysregulation.

According to embodiments of the present disclosure, the skin repair includes at least one of wound healing or skin regeneration.

According to the embodiments of the present disclosure, the ischemia-reperfusion injury includes at least one of ischemia-reperfusion injury during heart, lung, and kidney transplantation, or ischemia-reperfusion injury in muscles or brain tissue.

According to the embodiments of the present disclosure, the transplanted organ includes at least one of heart, liver, lung, or kidney.

A fifth aspect of the present disclosure provides a method for producing heterologous mitochondrial heterozygous cells. According to embodiments of the present disclosure, the method includes: co-incubating recipient cells containing autologous mitochondria with the mitochondrial transplantation therapy system of the first aspect, thereby enabling the heterologous mitochondria contained in the mitochondrial transplantation therapy system enter the recipient cells, and produce heterologous mitochondrial heterozygous cells. heterologous

A sixth aspect of the present disclosure provides a heterologous mitochondrial heterozygous cell. According to embodiments of the present disclosure, the heterologous mitochondrial heterozygous cell is prepared according to the method of the fifth aspect.

A seventh aspect of the present disclosure provides a method for repairing mitochondrial-damaged cells *in vitro*. According to embodiments of the present disclosure, the method includes: co-culturing the mitochondrial transplantation therapy system described in the first aspect with the mitochondrial-damaged cells, whereby normal mitochondria from the system enter the mitochondrial-damaged cells to effect repair.

An eighth aspect of the present disclosure provides a method for treating a disease associated with mitochondrial damage and/or repairing mitochondrial dysfunction. According to embodiments of the present disclosure, the method includes: administering the mitochondrial transplantation therapy system described in the first aspect and/or the medicament described in the second aspect to a subject.

According to the embodiments of the present disclosure, the mitochondrial damage includes the mitochondrial dysfunction and an mtDNA mutation.

According to the embodiments of the present disclosure, the mitochondrial functional damage includes both structural and metabolic damage.

According to the embodiments of the present disclosure, the mtDNA mutation refer to point mutations and deletion mutations.

According to the embodiments of the present disclosure, the mitochondrial dysfunction results from impairment of mitochondrial respiratory chain, aberrations in nuclear-encoded proteins, or alterations in mitochondrial physicochemical properties.

According to the embodiments of the present disclosure, the disease associated with the mitochondrial dysfunction includes, but are not limited to: nervous system disease, hearing impairment-related disease, optic neuropathy, myopathy, cardiac dysfunction, liver dysfunction, kidney dysfunction, pancreatic dysfunction, gastrointestinal dysfunction, metabolic disease, reproductive disease, or skeletal lesion attributable to such dysfunction.

According to the embodiments of the present disclosure, the nervous system disease includes at least one of neuronal development delay, Parkinson's disease, Alzheimer's disease, stroke, epilepsy, migraine, or amyotrophic lateral sclerosis.

According to the embodiments of the present disclosure, the hearing impairment-related disease includes at least one of deafness or sensorineural hearing loss.

According to the embodiments of the present disclosure, the optic neuropathy includes at least one of progressive external ophthalmoplegia, optic atrophy, or retinitis pigmentosa.

According to the embodiments of the present disclosure, the myopathy includes at least one of progressive muscle weakness and atrophy, sarcopenia, or exercise intolerance. Optionally, the cardiac dysfunction includes at least one of cardiomyopathy or a myocardial conduction defect.

According to the embodiments of the present disclosure, the liver dysfunction includes at least one of liver failure, liver damage, fatty liver, or liver cirrhosis.

According to the embodiments of the present disclosure, the liver dysfunction includes at least one of Fanconi syndrome, renal tubular acidosis, glomerulosclerosis, renal failure, or adrenal insufficiency.

According to the embodiments of the present disclosure, the pancreatic dysfunction includes at least one of diabetes mellitus or pancreatitis.

According to the embodiments of the present disclosure, the gastrointestinal dysfunction includes at least one of intestinal pseudo-obstruction or gastrointestinal motility disorder.

According to the embodiments of the present disclosure, the metabolic disease includes at least one of senility, obesity, hyperglycemia, dyslipidemia, insulin resistance, or cardiovascular disease.

According to the embodiments of the present disclosure, the reproductive disease includes at least one of premature ovarian failure or male infertility.

According to the embodiments of the present disclosure, the skeletal lesion includes at least one of kyphoscoliosis, short stature, or bone marrow failure.

A ninth aspect of the present disclosure provides the use of mitochondrial transplantation therapy system described in the first aspect, or the medicament according to the second aspect in the treatment of a disease associated with mitochondrial damage and/or in the repair of mitochondrial dysfunction.

According to embodiments of the present disclosure, the mitochondrial damage includes the mitochondrial dysfunction and mtDNA mutation.

According to the embodiments of the present disclosure, the mitochondrial functional damage encompasses impairment at both the structural and metabolic levels.

According to the embodiments of the present disclosure, the mtDNA mutation includes point mutation and deletion mutation.

According to the embodiments of the present disclosure, the mitochondrial dysfunction results from impairment of the mitochondrial respiratory chain, aberrations in nuclear-encoded proteins, or alterations in mitochondrial physicochemical properties.

According to the embodiments of the present disclosure, the disease associated with the mitochondrial dysfunction includes, but are not limited to: nervous system disease, hearing impairment-related disease, optic neuropathy, myopathy, cardiac dysfunction, liver dysfunction, kidney dysfunction, pancreatic dysfunction, gastrointestinal dysfunction, metabolic disease, reproductive disease, or skeletal lesion resulting from mitochondrial dysfunction.

According to the embodiments of the present disclosure, the nervous system disease includes, but are not limited to: neuronal development delay, Parkinson's disease, Alzheimer's disease, stroke, epilepsy, migraine, or amyotrophic lateral sclerosis.

According to the embodiments of the present disclosure, the hearing impairment-related disease includes at least one of deafness or sensorineural hearing loss.

According to the embodiments of the present disclosure, the optic neuropathy includes at least one of progressive external ophthalmoplegia, optic atrophy, or retinitis pigmentosa.

According to the embodiments of the present disclosure, the myopathy includes at least one of progressive muscle weakness and atrophy, sarcopenia, or exercise intolerance. Optionally, the cardiac dysfunction includes at least one of cardiomyopathy or a myocardial conduction defect.

According to the embodiments of the present disclosure, the liver dysfunction includes at least one of liver failure, liver damage, fatty liver, or liver cirrhosis.

According to the embodiments of the present disclosure, the liver dysfunction includes at least one of Fanconi syndrome, renal tubular acidosis, glomerulosclerosis, renal failure, or adrenal insufficiency.

According to the embodiments of the present disclosure, the pancreatic dysfunction includes at least one of diabetes mellitus or pancreatitis.

According to the embodiments of the present disclosure, the gastrointestinal dysfunction includes at least one of intestinal pseudo-obstruction or gastrointestinal motility disorder.

According to the embodiments of the present disclosure, the metabolic disease includes at least one of senility, obesity, hyperglycemia, dyslipidemia, insulin resistance, or cardiovascular disease.

According to the embodiments of the present disclosure, the reproductive disease includes at least one of premature ovarian failure or male infertility.

According to the embodiments of the present disclosure, the skeletal lesion includes at least one of kyphoscoliosis, short stature, or bone marrow failure.

Additional aspects and advantages of the present disclosure will be provided at least in part in the following description, or will become apparent at least in part from the following description, or can be learned from practicing of the present disclosure.

### BRIEF DESCRIPTION OF THE DRAWINGS

The above and/or additional aspects and advantages of the present disclosure will become more apparent and readily understood from the following description of embodiments provided in conjunction with the accompanying drawings.

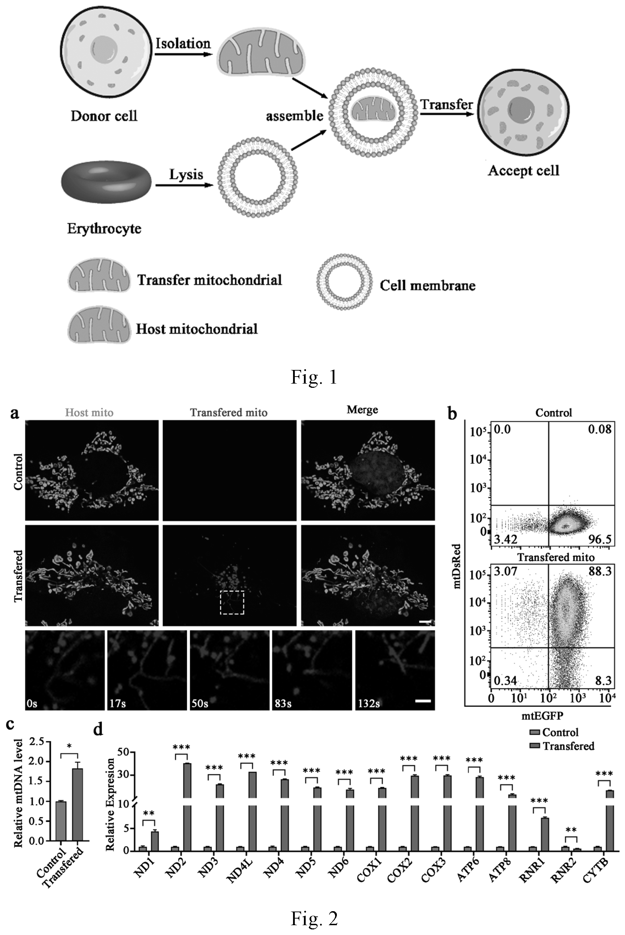

Fig. 1 Schematic diagram illustrating the construction of a mitochondrial transplantation therapy system.
Fig. 2 The mitochondria have been successfully transferred into cells and are functional post-transplantation. As described in Example 2 of the present disclosure, **a**, The morphology of transplanted mitochondria, and time-lapse imaging captures their dynamic changes within the cells; (Host mito: autologous mitochondria of the cells, Transfer mito: the transplanted mitochondria, scale: 5 µm, enlarged scale: 2 µm); **b**, Quantifies the transplantation efficiency of the mitochondrial transplantation therapy system of the present disclosure, as measured by flow cytometry; **c**, The relative level of mtDNA in receptor cells after mitochondrial transplantation 24 hours according to the present disclosure; **d**, The transcription levels of intracellular mtDNA after mitochondrial transplantation 24 hours according to the present disclosure.

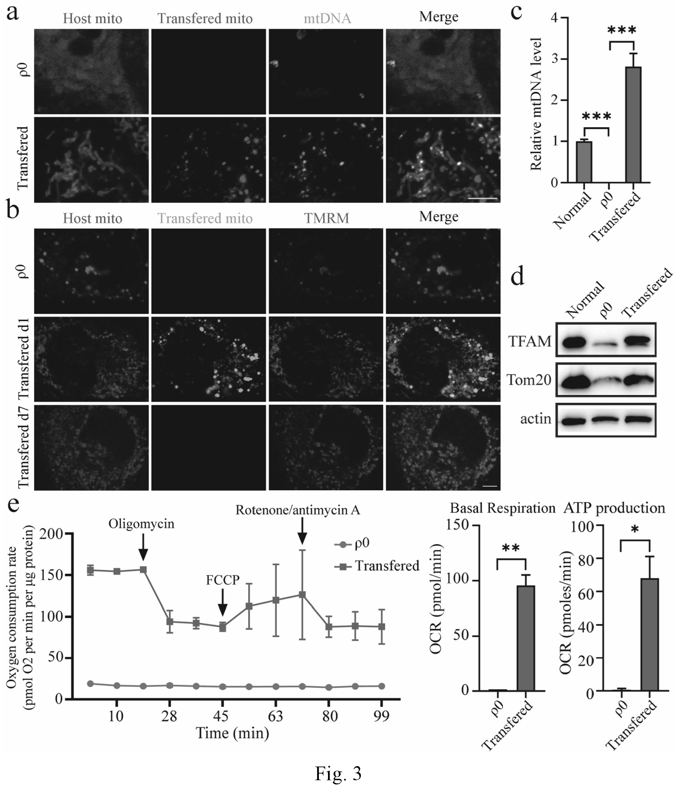

Fig. 3 Rescue the mitochondrial-devoid cells by mitochondrial transplantation. As set forth in Example 3 herein: **a**, Demonstrates the recovery effect of mtDNA levels in HeLa ρ0 cells following mitochondrial transplantation treatment; **b**, The recovery of mitochondrial membrane potential in HeLa ρ0 cells after the mitochondrial transplantation treatment; **c**, Quantifies the mtDNA copy number in HeLa ρ0 cells after the mitochondrial transplantation treatment; **d**, The mitochondrial proteins in rescued HeLa ρ⁰ cells after mitochondrial transplantation were determined by western blotting; **e**, The oxygen consumption rate in ρ⁰ cells after mitochondrial transplantation.

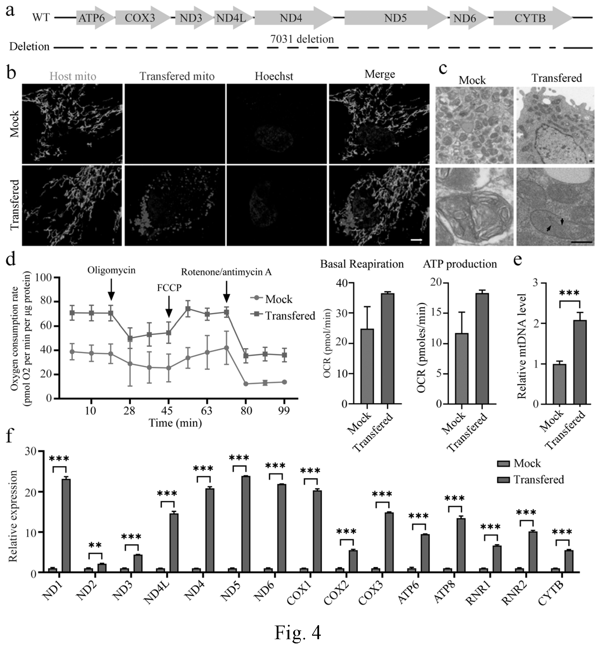

Fig. 4 Reduce the mitochondrial mutations in patient-derived cells. As in Example 4 of the present disclosure: **a**, The Schematic diagram represents the fragment deletion of mtDNA in human fibroblast GM04516, a patient-derived fibroblast cell line; **b**, Fluorescence image of exogenous mitochondria entering GM04516 cells following transplantation therapy system; **c**, Transmission electron microscopy reveals mitochondrial morphology in GM04516 cells following treatment with the mitochondrial transplantation therapy system of the present disclosure; **d**, The oxygen consumption rate in GM04516 cells after treatment with the mitochondrial transplantation therapy system of the present disclosure; **e**, Quantifies the mtDNA copy number in GM04516 cells after treatment with the mitochondrial transplantation therapy system of the present disclosure; **f**, Demonstrates quantitative changes in mtDNA transcription levels in GM04516 cells following treatment with the mitochondrial transplantation therapy system of the present disclosure.

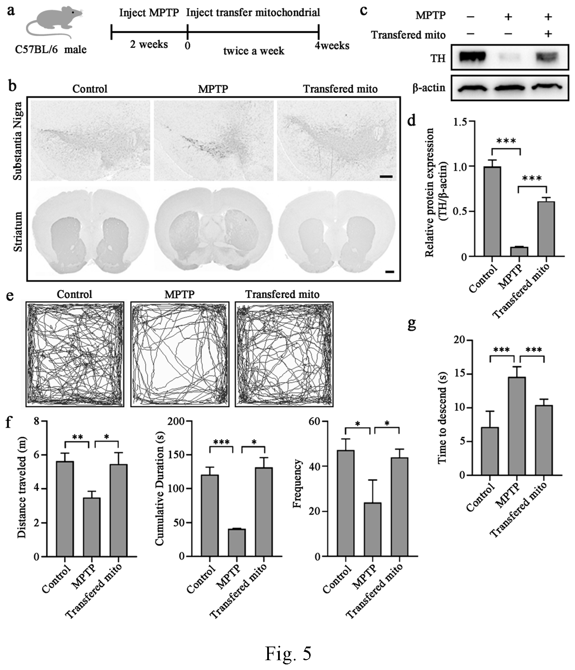

Fig. 5 Treatment of Parkinsonian mice with mitochondrial transplantation by intravenous injection in the examples 5. **a**, The Schematic diagram represents the mitochondrial transplantation in the treatment of Parkinsonian mice induced by MPTP. **b**, Recovery of tyrosine hydroxylase (TH)-positive neurons in the substantia nigra and striatum detected by immunohistochemistry after the mitochondrial transplantation treatment of the present disclosure; **c** and **d**, The TH expression in substantia nigra neurons detected by immunoblotting after mitochondrial transplantation; **e** and **f**, Motor function trajectory and statistical analysis of Parkinson's disease mice detected by open field test after 4 weeks of treatment with the mitochondrial transplantation therapy system of the present disclosure; **g**, Motor coordination ability of Parkinson's disease mice assessed by the pole test after 4 weeks of treatment with the mitochondrial transplantation therapy system of the present disclosure.

### DETAILED DESCRIPTION

Embodiments of the present disclosure will be described in detail below. The embodiments described below are exemplary and are intended only to explain the present disclosure, which should not be construed as a limitation to the present disclosure.

It should be noted that the terms "first" and "second" are only used for descriptive purposes, rather than indicating or implying relative importance or implicitly indicating the number of indicated technical features. Therefore, the features defined with "first" and "second" may include one or more of these features explicitly or implicitly. Further, in the description of the present disclosure, unless otherwise specified, "plurality of" means two or more.

In the present disclosure, endpoints and any value of the ranges shall not be limited to the exact range or value, and those ranges or values should be understood to include values close to those ranges or values. For numerical ranges, endpoints of respective ranges, an endpoint and individual point value of respective ranges, and individual point values can be combined with each other to obtain one or more new numerical ranges, which should be deemed to be specifically disclosed herein.

It should be noted that, unless otherwise indicated, scientific and technical terms used herein have the meanings commonly understood by those skilled in the art. In addition, the terms related in protein, nucleic acid chemistry, and molecular biology, as well as the laboratory operating procedures used herein are terms and conventional procedures widely used in the corresponding fields. For example, the standard recombinant DNA and molecular cloning technologies used in the present disclosure are well known to those skilled in the art and are described more comprehensively in the following literature: Sambrook, J., Fritsch, E.F., and Maniatis, T., Molecular Cloning: A Laboratory Manual; Cold Spring Harbor Laboratory Press: Cold Spring Harbor, 1989 (hereinafter referred to as "Sambrook").

As used herein, the term "comprise" or "include" is an open-ended expression, that is, including the contents indicated by the present disclosure, but not excluding content in other aspects.

As used herein, the terms "optionally" or "optional" generally mean that the event or situation described subsequently may but not necessarily occur, and the description includes the situation in which the event or situation occurs and the situation in which the event or situation does not occur.

As used herein, the term "and/or" encompasses all combinations of items connected by the term, and each combination shall be deemed to have been individually listed herein. For example, "A and/or B" encompasses "A," "A and B," and "B." For example, "A, B, and/or C" encompasses "A," "B," "C," "A and B," "A and C," "B and C," and "A and B and C."

According to a specific embodiment of the present disclosure, a mitochondrial transplantation therapy system is provided. The system includes: isolated mitochondria; and a transfer vector. The system is obtained by assembling the mitochondria with the transfer vector. The transfer vector includes at least one of an artificial lipid vesicle, a cell-derived vesicle, or an exosome.

It should be noted that, the mitochondria and transfer vector may be assembled by various methods. Exemplary methods include direct mixing and co-incubating, or adding other reagents that promote the entry or association of mitochondria with transfer vector.

In the present disclosure, the terms "healthy" or "normal" mitochondria refer to isolated mitochondria possessing normal structure and function, and which are free from mtDNA damage. The mtDNA damage includes, but is not limited to, mitochondrial structural damage, mitochondrial metabolic damage, mtDNA mutation, and mtDNA deletion.

There is no particularly limited on a source of the isolated mitochondria contained in the mitochondrial transplantation therapy system. For example, they are isolated from a mammalian cell. In some embodiments, the mammalian cell may be a human cell.

It should be noted that, in preparing the mitochondrial transplantation therapy system, the mass ratio of the transfer vector (e.g., the artificial lipid vesicle) to the mitochondria is not critical. In a preferred approach, an excess of mitochondria is provided relative to vesicles to maximize the encapsulation efficiency. In an exemplary preparation method, the transfer vector and mitochondria are combined at a mass ratio of 1:2. The transfer vector (e.g., the artificial lipid vesicle) is a biodegradable component, ensuring biocompatibility and no interference with the therapeutic effect. The assembled transplanted mitochondria particles are relatively large and can be precipitated to the bottom of the centrifuge tube by centrifugation. Following centrifugation, the supernatant containing unencapsulated mitochondria is discarded. According to a specific embodiment, the present disclosure provides a medicament including the above-described mitochondrial transplantation therapy system. This medicament can be used to repair the mitochondrial damage and to treat disease associated with mitochondrial dysfunction.

According to a specific embodiment of the present disclosure, the damaged mitochondria is attributed to damage in mitochondrial DNA (mtDNA). Cells with mitochondrial dysfunction include mtDNA-depleted (ρ⁰) cells, cells with large-fragment mtDNA deletions, or cells with mtDNA point mutations. According to another specific embodiment of the present disclosure, the cells with mitochondrial dysfunction may also result from mitochondrial respiratory chain damage, an abnormality of nuclear-encoded proteins, or a change in physicochemical properties (e.g., membrane potential).

According to a specific embodiment of the present disclosure, the mitochondrial transplantation therapy system provided herein, and the medicament containing the mitochondrial transplantation therapy system can be used to treat the disease associated with the mitochondrial dysfunction. Disease associated with the mitochondrial dysfunction includes at least one of a nervous system disease, hearing impairment-related disease, optic neuropathy, myopathy, cardiac dysfunction, liver dysfunction, kidney dysfunction, pancreatic dysfunction, gastrointestinal dysfunction, metabolic disease, reproductive disease, or skeletal lesion. However, it is understood that the types of diseases associated with the mitochondrial dysfunction are not limited to those listed herein, and the present disclosure encompasses any other diseases known in the art to be caused by the mitochondrial dysfunction. According to a specific embodiment of the present disclosure, the mitochondrial transplantation therapy system is used for skin repair, including at least one of wound healing or skin regeneration.

According to a specific embodiment of the present disclosure, the mitochondrial transplantation therapy system is used to alleviate ischemia-reperfusion injury during organ transplantation. The application includes at least one of ischemia-reperfusion injury during heart, lung, and kidney transplantation, as well as ischemia-reperfusion injury in muscles or brain tissue.

According to a specific embodiment of the present disclosure, the mitochondrial transplantation therapy system is used to prolong a preservation duration of a transplanted organ. The transplanted organ includes at least one of heart, liver, lung, or kidney transplantation.

According to a specific embodiment of the present disclosure, the mitochondrial transplantation therapy system is used to mitigate aging, organ degeneration, and diseases associated with aging, such as at least one of a neurodegenerative disease or cardiovascular disease.

According to a specific embodiment of the present disclosure, the mitochondrial transplantation therapy system is used for at least one of diseases such as weight loss and organ metabolic regulation.

According to an embodiment of the present disclosure, the present disclosure provides a method for generating cells harboring both autologous and heterologous mitochondria. The method includes: co-incubating the mitochondrial transplantation therapy system as described herein with recipient cells. The heterologous mitochondria contained in the mitochondrial transplantation therapy system enter the recipient cells, to obtain the heterologous mitochondrial heterozygous cells.

It should be noted that, the source of healthy mitochondria for the system is not restricted. The mitochondria may be autologous (derived from the same cell type as the target) or heterologous (derived from a different cell type). Specifically, the donor cells providing the healthy mitochondria and the recipient cell may originate from different organs, tissues, or species. Suitable donor cells encompass both primary cells or established cell lines.

It should be noted that, although the inventors have mainly verified in the examples that the new mitochondrial transplantation therapy system provided in the present disclosure can repair mtDNA-depleted cells and in Parkinson's disease mice caused by mitochondrial dysfunction. The underlying innovation is embodied in the design of the system: the system reliably preserves encapsulated mitochondrial structure and function, enables efficient cellular entry, the specific mitochondrial transplantation therapy system provided in the present disclosure can maintain the morphology and function of the encapsulated healthy mitochondria, efficiently enter the recipient cell, and ultimately restores mitochondrial activity in recipient cells. Therefore, based on its mechanism of action, the mitochondrial transplantation therapy system provided in the present disclosure holds therapeutic potential for a broad spectrum of diseases associated with mitochondrial dysfunction. This includes, but is not limited to, nervous system disease, hearing impairment-related disease, optic neuropathy, myopathy, cardiac dysfunction, liver dysfunction, kidney dysfunction, pancreatic dysfunction, gastrointestinal dysfunction, metabolic disease, reproductive disease, or skeletal lesion. In addition, previous studies have reported that mitochondrial transplantation can also be used for skin repair, alleviating ischemia-reperfusion injury associated with organ transplantation, prolonging a preservation duration of the transplanted organ, treating or alleviating organ metabolic dysregulation, etc. These reported applications underscore the broad potential utility of the mitochondrial transplantation therapy system disclosed herein in addressing these and related conditions.

According to preferred embodiments of the present disclosure, experimental evidence shows that the transplantation of mitochondria from diverse sources into various recipient cell types is feasible and effective. For example, mitochondria isolated from HeLa or Cos7 cells can be successfully transplanted into and remain functional within recipient cells such as HeLa, Cos7, U20S, and 3T3 cells, demonstrating functional compatibility across different cell types within the same species. Due to the heterogeneity of mitochondria, it is preferable to source transplantation mitochondria from cells that are identical or similar to the cells to the recipient cells. Mitochondrial quantity and metabolic activity vary significantly among different cell types. Therefore, preference should be given to the selection of cells with a high degree of similarity as mitochondrial donors.

The solutions of the present disclosure will be illustrated with examples. Those skilled in the art can understand that the following examples are only used to illustrate the present disclosure and should not be regarded as limiting the scope of the present disclosure. If the specific technologies or conditions are not specified in the examples, experimental procedures were performed using conventional techniques in the art or according to the manufacturer's instructions. All reagents and instruments not explicitly indicating their source are commercially available standard products.

### Example 1: Construction of New Mitochondrial Transplantation Therapy System

The mitochondrial transplantation therapy system was constructed according to the following methods:

### 1. Preparation of Artificial Lipid Vesicles

The C57BL/6J mice were obtained from the Experimental Animal Center and anesthetized with 1.25% avertin (0.2 mL/10 g) via intraperitoneal injection. Following anesthesia induction, blood was collected from the orbital sinus using a glass capillary tube. blood was collected from the orbital sinus using a glass capillary tube. Approximately 100 - 200 µL of blood was collected per mouse. An appropriate amount of anticoagulant was added to the collection tube to prevent blood coagulation. The blood sample was centrifuged at 800 g for 10 minutes, and the supernatant was gently aspirated to obtain dark red cell pellets. The blood cell pellets were resuspended in an appropriate amount of pre-cooled PBS by gentle pipetting. The cells were filtered through a 0.45 µm filter to remove cell aggregates. The cell suspension was centrifuged at 800 g for 10 minutes. The supernatant was discarded, and then an appropriate amount of pre-cooled PBS was added, followed by centrifugation. This operation was repeated 2 to 3 times. An appropriate amount of pre-cooled 25% PBS solution was added to the cell pellets. The mixture was incubated with rotation for 15 - 30 minutes, followed by centrifuged at 800 g for 10 minutes and the supernatant was discarded. An appropriate amount of pre-cooled PBS solution was added, followed by centrifugation, and this operation was repeated 2 to 3 times. Subsequently, the artificial lipid vesicles were aliquoted and stored at - 80°C.

### 2. Mitochondrial Extraction

For the target of interest, any cells of the corresponding species, including primary cells or cell lines, were selected, cultured and expanded *in vitro*. Upon reaching approximately 5×10⁷ cells, the cells were digested using 0.25% trypsin and collected, then centrifuged at 300 g for 3 minutes. The supernatant was discarded, and DPBS solution was added to resuspend the cells. The mixture was centrifuged at 300 g for 3 minutes, and the supernatant was carefully aspirated. An appropriate amount of mitochondrial isolation buffer was added to resuspend the pellet, followed by incubation on ice for 10 to 20 minutes. The cells were homogenized on ice with 25 to 35 times on ice using 5 mL syringe. The homogenate was then centrifuged at 800 g for 5 minutes in 4°C. Subsequently, the supernatant was carefully aspirated into a new tube, avoiding the pelleted nuclei and cellular debris, and subjected to refrigerated centrifugation at 12,000 g for 5 minutes in 4 °C. The supernatant was discarded, and crude mitochondria pellets were initially obtained. For the construction of the transplantation system, the mitochondria pellets were required to be stored on ice and used immediately after extraction.

The extracted mitochondria were resuspended and combined with the artificial lipid vesicles, then incubated with continuous gentle agitation using a magnetic stirrer for 1 hour to facilitate encapsulation. Subsequently, the mixed solution was centrifuged at 800 g for 5 minutes, and the supernatant was discarded to obtain the encapsulated mitochondrial transplantation therapy system, as illustrated in Fig. 1.

### Example 2: The mitochondria have been successfully transferred into cells and are functional post-transplantation

### 1. Process of Mitochondrial Transplantation into Cells

The HeLa cells were seeded into a 6 cm cell culture dishes at a density of 3 - 5×10⁵ cells per well, and cultured for 12 - 24 hours to allow adhesion. The encapsulated mitochondria (prepared in Example 1) were resuspended in DMEM culture medium containing penicillin and streptomycin (10% fetal bovine serum, 1% MEM NEAA, and GlutaMax), and then added to the culture plate. After co-culturing for 6 - 12 hours, cells were washed 3 times with DPBS, and culture medium containing penicillin and streptomycin was added to continue culturing. Cell samples were collected, and genome DNA and RNA were extracted in parallel using commercial kits according to the manufacturers' instructions. Finally, the expression levels of mitochondria-related genes were quantified to assess the transcriptional effects of mitochondrial transplantation.

### 2. Mitochondrial Transplantation Efficiency and Mitochondrial Morphology

Following encapsulation by the cell membrane vector, the mitochondria were internalized into recipient cell via endocytosis. After membrane fusion, the mitochondria were subsequently released into the cell. For visualization, the mitochondria were pre-labeled with the fluorescent proteins EGFP and DsRed, respectively. After the cells was expanded, the fluorescently labeled mitochondria were extracted. These labeled mitochondria were then transplanted into recipient cells. Their intracellular persistence and viability were subsequently assessed using live-cell imaging techniques. HeLa cells were selected for the transfection experiment, DsRed-labeled mitochondria were extracted from HeLa mtDsRed cells and transplanted into HeLa mtGFP cells as shown in Fig. 2a. Live-cell imaging revealed that most of the transplanted mitochondria were spherical, while a subset recovered an elongated, linear structure. Time-lapse imaging revealed that the transplanted mitochondria were dynamic, with tubular and spherical forms interconverting, indicates that the transplanted mitochondria were viable and functional within the cells. To detect the transplantation efficiency of the mitochondrial transplantation therapy system, flow cytometry was further employed to measure the transplantation efficiency of the exogenous mitochondria as shown in Fig. 2b. The system exhibited remarkable transplantation efficiency, as evidenced by the presence of exogenous mitochondria in >90% of cells within 24 hours. This underscores its potential for applications requiring swift and widespread mitochondrial transfer.

Mitochondrial abundance varies considerably across cell types, being particularly high in metabolically active cells and tissues. Mitochondria harbor their own DNA (mtDNA), the quantity and quality are crucial for maintaining mitochondrial functions and cellular homeostasis. Consequently, measuring mtDNA copy number serves as a practical and preliminary method for assessing changes in mitochondrial abundance. A significant increase in intracellular mtDNA copy number was confirmed following mitochondrial transplantation. This was quantified by qPCR analysis of genomic DNA isolated from cell samples at the 24-hour time point (Fig. 2c). To explore the impact of mitochondrial transplantation on mtDNA transcription, the transcript levels of all 13 proteins-coding genes and 2 rRNAs genes encoded by mtDNA were detected by qPCR. Total RNA were extracted 24 hours after the mitochondrial transplantation, and the relative expression level of mtDNA transcription was detected. As illustrated Fig. 2d, the transcription level of mtDNA encoded was markedly upregulated at 24 hours after the mitochondrial transplantation. In summary, the mitochondrial transplantation therapy system can effectively transplant mitochondria into cells, where they achieve successful functional integration and restore bioenergetic capacity.

### Example 3: Repair of Mitochondrial Functions in mtDNA-Depleted Cells by the Mitochondrial Transplantation Therapy System

### 1. Construction and Identification of mitochondrial DNA-Depleted Cells

To investigate the reparative potential of the mitochondrial transplantation therapy system, mtDNA-depleted cells (ρ0 cells) were constructed as a model of severe mitochondrial damage. HeLa cells were treated with ethidium bromide (EB), and passaged cells were collected. The depletion of mtDNA was rigorously monitored by periodic assessment of both mtDNA copy number (via qPCR of genomic DNA) and the expression of mtDNA-encoded proteins, until mtDNA was nearly undetectable. These validated ρ⁰ cells were used to explore the system's capacity to restore mitochondrial functions.

### 2. Repair of mitochondrial functions in ρ0 Cells by mitochondrial transplantation therapy system

Mitochondria labeled with EGFP or DsRed were extracted and assembled with red blood cell-derived artificial lipid vesicles according to the method described in Example 1. After the fluorescently labeled mitochondria was transplanted into recipient cells, the internalization, viability, and persistence of the exogenous mitochondria the exogenous mitochondria were subsequently assessed using live-cell imaging. After transferring the mitochondria into ρ0 cells with the disclosed transplantation system 24 hours, Picogreen staining confirmed that mitochondria in untreated ρ0 cells basically contained no mtDNA. In contrast, post-treatment analysis revealed that most of the mitochondria in ρ0 cells had successfully incorporated mtDNA, and the morphology of mitochondria in ρ0 cells recovered from a fragmented, abnormal state to the elongated, linear shape characteristic of healthy mitochondria (as illustrated in Fig. 3a). At the same time, mitochondrial membrane potential was assessed using the fluorescent dye TMRM. As illustrated in Fig. 3b, 1 to 7 days after transplantation, restoration of both the morphology and membrane potential of mitochondria was observed from 1 to 7 days post-transplantation, indicating a recovery of mitochondrial metabolic function. Consistent with this functional recovery, qPCR analysis confirmed a significant restoration of the relative mtDNA copy number as early as 24 hours after treatment (Fig. 3c). To further evaluate mitochondrial recovery, we assessed the protein levels of TFAM (critical for mtDNA maintenance) and Tom20 (essential for protein import). Their elevated expression at day 7 (Fig. 3d) demonstrates that the transplanted mitochondria not persisted but also activated the host cell's program for mitochondrial biogenesis and functional assembly. In order to definitively assess the restoration of core mitochondrial function, cellular respiration was analyzed using the Seahorse XF Analyzer, the gold-standard assay for mitochondrial oxidative phosphorylation. The results showed that after 24 hours of mitochondrial transplantation treatment of the present disclosure, the oxidative phosphorylation metabolism level of ρ0 cells was effectively restored, and the ATP production was significantly improved (Fig. 3e). In summary, the mitochondrial transplantation therapy system of the present disclosure can effectively repair the mitochondrial functions in mtDNA-depleted cells.

### Example 4: Reduce Mitochondrial mutations in patient-derived Cells by Mitochondrial Transplantation Therapy System

Mitochondrial DNA (mtDNA) mutations and deletions arising during replication can compromise mitochondrial function and lead to disease. To further evaluate the therapeutic scope of the system, we employed GM04516 cells (purchased from the Coriell Institute), which harbor a pathogenic 7031bp deletion in mtDNA(Fig. 4a). This model was used to explore whether the mitochondrial transplantation therapy system could rescue the associated mitochondrial dysfunction.

DsRed-labeled mitochondria were extracted from the primary human skin fibroblast GZF2 (derived from the Guangzhou Institute of Biomedicine and Health), and then transferred into GM04516 cells using the transplantation therapy system of the present disclosure (the same assembled transplantation therapy system as that in Example 3). Mitochondria were labeled with the MitoTracker deep red after treating with the transplantation therapy system 24 hours. It can be observed that majority of the transplanted mitochondria within GM04516 cells exhibited a spherical morphology, while a subset had reacquired an elongated, linear shape (Fig.4b). To assess ultrastructural restoration, GM04516 cells were processed for transmission electron microscopy (TEM) 7 days post-transplantation. As shown in Fig. 4c, untreated control cells (with large-fragment mtDNA deletions) exhibited aberrant mitochondrial ultrastructure, including disorganized cristae. In contrast, cells treated with the mitochondrial transplantation therapy system displayed a clear restoration of normal morphology, with well-defined cristae distinctly visible. These results demonstrate that the system not only delivers healthy mitochondria but also facilitates the repair of damaged cellular ultrastructure.

The large-fragment mtDNA deletion in GM04516 cells impairs the production of respiratory chain complexes, leading to a decrease in cellular oxidative phosphorylation (OXPHOS). To determine whether mitochondria transplantation could rescue this bioenergetic deficit, the oxygen consumption rate (OCR) of GM04516 cells was assessed 24 hours post-treatment. Seahorse metabolism analysis revealed that the basal respiration level of GM04516 cells was significantly increased after treatment with the mitochondrial transplantation of the present disclosure (Fig. 4d). In addition, mitochondria ATP production and the maximal respiratory capacity of the cells were significantly enhanced following treatment. The restoration of oxidative phosphorylation (OXPHOS) boosts the cellular bioenergetic capacity and metabolic flexibility, enabling cells to better withstand external stressors. Collectively, the results of cell metabolism detection indicated that the mitochondrial transplantation therapy system of the present disclosure can effectively rescues the core bioenergetic function in cells harboring large-fragment mtDNA deletions, which help cells maintain a stable energy supply.

Following treatment with the mitochondrial transplantation therapy system of the present disclosure, the inventors detected an increase in mtDNA copy number of GM04516 cells, as quantified by qPCR analysis (Fig. 4e). (by panel e in Fig. 4). To assess the functional impact of the restored mtDNA, transcriptional was analyzed by qPCR. As illustrated in Fig. 4f, total mtDNA-encoded transcript levels (encompassing mRNAs and rRNAs) were significantly elevated 24 hours post-transplantation. Critically, transcripts of genes located within the deleted region - including *ATP6*, *ND3*, *ND4L*, *ND4*, *ND5*, *ND6*, and *CYTB* were all significantly increased, indicating that the mitochondrial transplantation therapy system of the present disclosure can effectively improve mitochondrial functions in cells with large mitochondrial DNA fragment deletions.

### Example 5: Repair of Symptoms in Parkinson's Disease Mice Caused by Mitochondrial Dysfunction by Mitochondrial Transplantation Therapy System

### 1. Construction of a Parkinson's Disease Mouse Model

C57BL/6J mice (6 - 8 weeks old) were purchased from Guangdong Yaokang Biotechnology Co., Ltd. Following an acclimatization period, the mice were divided into cages with 5 - 6 mice per cage. They were housed in an SPF facility under controlled conditions: 22 °C, 30% relative humidity, a 12-hour light/dark cycle, with ad libitum access to food and water. Mice were ear-tagged and body weights were recorded. At approximately 20 g (6-8 weeks of age), mice were anesthetized with via intraperitoneal injection of 1.25% avertin (0.2 mL/10 g). The mice's toes were pinched to confirm whether they were fully anesthetized. To induce Parkinsonian symptoms, mice received daily intraperitoneal injections of MPTP (2.5 mg/kg body weight) for 5 consecutive days. Animal health was closely monitored post-injection, and any mouse exhibiting severe distress was humanely euthanized. Approximately 14 days after the initial MPTP injection, the mice were euthanized, and brain tissues were collected for validation of the Parkinson's disease model.

### 2. Mitochondrial Transplantation Therapy System Repairs Behavioral Deficits in Parkinson's Disease Mice Caused by Mitochondrial Dysfunction

Parkinson's disease is a neurodegenerative disorder. Its pathology in this model is mediated by MPP⁺, the neurotoxic metabolite of MPTP *in vivo*, which selectively damage the mitochondria of dopaminergic neurons. Following a two weeks of MPTP treatment (Fig. 5a), mice developed characteristic Parkinsonian motor symptoms, including bradykinesia and tremors. Following a four weeks of treatment with the mitochondrial transplantation therapy system of the present disclosure (obtained by assembling mouse fibroblast-derived mitochondria with mouse red blood cells according to the method in Example 1). The mice were euthanized, and brain tissues were harvested, fixed, and sectioned. To evaluate the therapeutic efficacy, the expression of tyrosine hydroxylase (TH) - a marker of dopaminergic neurons was assessed in both substantia nigra and striatum, characterize the potential repair of these neuronal populations. As illustrated in Fig. 5b, MPTP treatment led to a marked reduction in TH-positive dopaminergic neurons at both substantia nigra and striatum. In contrast, treatment with the mitochondrial transplantation therapy system of the present disclosure, the number of TH-containing dopaminergic neurons in substantia nigra and striatum was restored. Protein was extracted from the substantia nigra, and the expression of TH was detected by immunoblotting. It was clearly seen that, the expression of TH levels significantly decreased after MPTP treatment. However, treatment with the mitochondrial transplantation therapy system led to a significant upregulation of TH expression (Fig. 5c, d), indicating restoration of this key dopaminergic marker at the molecular level.

To evaluate the restoration of motor function, Parkinson's disease mice were subjected to open field and pole climbing tests. The results indicated that MPTP-treated mice exhibited significantly reduced total distance and decreased frequency of entering the central area of the open field, indicating motor deficits and anxiety-like behavior. Importantly, treatment with the mitochondrial transplantation therapy system of the present disclosure markedly improved these behavioral parameters, as quantified in Fig. 5e, f, demonstrating a recovery of motor function. As illustrated in Fig. 5g, MPTP treatment markedly prolonged the time required for mice to descend the pole. In contrast, treatment with the mitochondrial transplantation therapy system of the present disclosure reduced this descent time, indicating a robust recovery of coordinated motor function. In summary, the mitochondrial transplantation therapy system can alleviate the symptoms of Parkinson's disease mice and exhibit a certain therapeutic effect.

Throughout this specification, reference to terms such as "an embodiment", "some embodiments", "an example", "a specific example", or "some examples" indicate that a particular feature, structure, material, or characteristic described in connection with the embodiment or example is included in at least one embodiment or example of the present disclosure. The schematic expressions of the above terms throughout this specification are not necessarily referring to the same embodiment or example. Further, the particular features, structures, materials, or characteristics may be combined in any suitable manner in one or more embodiments or examples. In addition, different embodiments or examples and features of different embodiments or examples described in the specification may be combined and integrated by those skilled in the art without mutual contradiction.

Although embodiments of the present disclosure have been shown and described above, it should be understood that above embodiments are merely exemplary, and cannot be construed to limit the present disclosure. For those skilled in the art, changes, modifications, substitutions, and variations can be made to the embodiments without departing from the scope of the present disclosure.

## Claims

1. A mitochondrial transplantation therapy system, comprising:
isolated mitochondria; and
a transfer vector,
wherein the mitochondrial transplantation therapy system comprises mitochondria assembled with a transfer vector, and wherein the transfer vector is selected from the group consisting of an artificial lipid vesicle, a cell-derived vesicle, an exosome, and any combination thereof.

2. The mitochondrial transplantation therapy system according to claim 1, wherein the transfer vector comprises a vesicle derived from red blood cells, selected from a native red blood cell membrane vesicle or a reconstituted lipid vesicle comprising red blood cell membrane components.

3. The mitochondrial transplantation therapy system according to claim 1, wherein the mitochondria are isolated from a mammalian cell.

4. A medicament, comprising the mitochondrial transplantation therapy system according to any one of claims 1 to 3.

5. Use of the mitochondrial transplantation therapy system according to any one of claims 1 to 3, or the medicament according to claim 4, in the manufacture of a medicament for repairing mitochondrial damage.

6. The use according to claim 5, wherein the mitochondrial damage comprises mitochondrial functional damage and an mtDNA mutation;
optionally, the mitochondrial functional damage comprises mitochondrial structural damage and mitochondrial metabolic damage;
optionally, the mtDNA mutation comprises an mtDNA point mutation and an mtDNA deletion mutation.

7. Use of the mitochondrial transplantation therapy system according to any one of claims 1 to 3, or the medicament according to claim 4, in the manufacture of a medicament for treating a disease associated with mitochondrial dysfunction.

8. The use according to claim 7, wherein the mitochondrial dysfunction is caused by a mitochondrial respiratory chain damage, an abnormality of nuclear-encoded mitochondrial proteins, or a change in mitochondrial physicochemical properties;
optionally, the disease associated with the mitochondrial dysfunction is selected from the group consisting of: a nervous system disease, hearing impairment-related disease, optic neuropathy, myopathy, cardiac dysfunction, liver dysfunction, kidney dysfunction, pancreatic dysfunction, gastrointestinal dysfunction, metabolic disease, reproductive disease, or skeletal lesion caused by the mitochondrial dysfunction;
optionally, the nervous system disease comprises at least one of neuronal development delay, Parkinson's disease, Alzheimer's disease, stroke, epilepsy, migraine, or amyotrophic lateral sclerosis;
optionally, the hearing impairment-related disease comprises at least one of deafness or sensorineural hearing loss;
optionally, the optic neuropathy comprises at least one of progressive external ophthalmoplegia, optic atrophy, or retinitis pigmentosa;
optionally, the myopathy comprises at least one of progressive muscle weakness and atrophy, sarcopenia, or exercise intolerance;
optionally, the cardiac dysfunction comprises at least one of cardiomyopathy or myocardial conduction defect;
optionally, the liver dysfunction comprises at least one of liver failure, liver damage, fatty liver, or liver cirrhosis;
optionally, the liver dysfunction comprises at least one of Fanconi syndrome, renal tubular acidosis, glomerulosclerosis, renal failure, or adrenal insufficiency;
optionally, the pancreatic dysfunction comprises at least one of diabetes mellitus or pancreatitis;
optionally, the gastrointestinal dysfunction comprises at least one of intestinal pseudo-obstruction or gastrointestinal motility disorder;
optionally, the metabolic disease comprises at least one of senility, obesity, hyperglycemia, dyslipidemia, insulin resistance, or cardiovascular disease;
optionally, the reproductive disease comprises at least one of premature ovarian failure or male infertility; and
optionally, the skeletal lesion comprises at least one of kyphoscoliosis, short stature, or bone marrow failure.

9. Use of the mitochondrial transplantation therapy system according to any one of claims 1 to 3, or the medicament according to claim 4, in the manufacture of the following medicaments:
(1) a medicament for skin repair;
(2) a medicament for alleviating ischemia-reperfusion injury associated with organ transplantation;
(3) a medicament for prolonging a preservation duration of a transplanted organ; and
(4) a medicament for treating or alleviating organ metabolic dysregulation,
optionally, the skin repair comprises at least one of wound healing or skin regeneration;
optionally, the ischemia-reperfusion injury comprises at least one of ischemia-reperfusion injury during heart, lung, and kidney transplantation, or ischemia-reperfusion injury in muscles or brain tissue; and
optionally, the transplanted organ comprises at least one of heart, liver, lung, or kidney.

10. A method for producing heterologous mitochondrial heterozygous cells, the method comprising:
co-incubating the mitochondrial transplantation therapy system according to any one of claims 1 to 3 with recipient cells containing autologous mitochondria, wherein heterologous mitochondria contained in the mitochondrial transplantation therapy system enter the recipient cells, to obtain the heterologous mitochondrial heterozygous cells.

11. A heterologous mitochondrial heterozygous cell, being obtained by the method according to claim 10.

12. A method for repairing mitochondrial-damaged cells *in vitro*, the method comprising:
co-culturing the mitochondrial transplantation therapy system with the mitochondrial-damaged cells according to any one of claims 1 to 3, wherein normal mitochondria contained in the mitochondrial transplantation therapy system enter the mitochondrial-damaged cells, to repair the mitochondrial-damaged cells.

13. A method for treating a disease associated with mitochondrial damage and/or repairing mitochondrial dysfunction, the method comprising:
administering the mitochondrial transplantation therapy system according to any one of claims 1 to 3 and/or the medicament according to claim 4 to a subject.

14. The method according to claim 13, wherein the mitochondrial damage comprises the mitochondrial dysfunction and/or an mtDNA mutation;
optionally, the mitochondrial functional damage comprises mitochondrial structural damage and mitochondrial metabolic damage;
optionally, the mtDNA mutation comprises an mtDNA point mutation and an mtDNA deletion mutation;
optionally, the mitochondrial dysfunction is caused by a mitochondrial respiratory chain damage, an abnormality of nuclear-encoded mitochondrial proteins, or a change in mitochondrial physicochemical properties;
optionally, the disease associated with the mitochondrial dysfunction is selected from the group consisting of: a nervous system disease, hearing impairment-related disease, optic neuropathy, myopathy, cardiac dysfunction, liver dysfunction, kidney dysfunction, pancreatic dysfunction, gastrointestinal dysfunction, metabolic disease, reproductive disease, or skeletal lesion caused by the mitochondrial dysfunction;
optionally, the nervous system disease comprises at least one of neuronal development delay, Parkinson's disease, Alzheimer's disease, stroke, epilepsy, migraine, or amyotrophic lateral sclerosis;
optionally, the hearing impairment- related disease comprises at least one of deafness or sensorineural hearing loss;
optionally, the optic neuropathy comprises at least one of progressive external ophthalmoplegia, optic atrophy, or retinitis pigmentosa;
optionally, the myopathy comprises at least one of progressive muscle weakness and atrophy, sarcopenia, or exercise intolerance;
optionally, the cardiac dysfunction comprises at least one of cardiomyopathy or a myocardial conduction defect;
optionally, the liver dysfunction comprises at least one of liver failure, liver damage, fatty liver, or liver cirrhosis;
optionally, the liver dysfunction comprises at least one of Fanconi syndrome, renal tubular acidosis, glomerulosclerosis, renal failure, or adrenal insufficiency;
optionally, the pancreatic dysfunction comprises at least one of diabetes mellitus or pancreatitis;
optionally, the gastrointestinal dysfunction comprises at least one of intestinal pseudo-obstruction or gastrointestinal motility disorder;
optionally, the metabolic disease comprises at least one of senility, obesity, hyperglycemia, dyslipidemia, insulin resistance, or cardiovascular disease;
optionally, the reproductive disease comprises at least one of premature ovarian failure or male infertility; and
optionally, the skeletal lesion comprises at least one of kyphoscoliosis, short stature, or bone marrow failure.

15. Use of the mitochondrial transplantation therapy system according to any one of claims 1 to 3, or the medicament according to claim 4, in the treatment of a disease associated with mitochondrial damage and/or in the repair of mitochondrial dysfunction.

16. The use according to claim 15, wherein the mitochondrial damage comprises the mitochondrial dysfunction and/or an mtDNA mutation;
optionally, the mitochondrial functional damage comprises mitochondrial structural damage and mitochondrial metabolic damage;
optionally, the mtDNA mutation comprises an mtDNA point mutation and an mtDNA deletion mutation;
optionally, the mitochondrial dysfunction is caused by a mitochondrial respiratory chain damage, an abnormality of nuclear-encoded mitochondrial proteins, or a change in mitochondrial physicochemical properties;
optionally, the disease associated with the mitochondrial dysfunction is selected from the group consisting of: a nervous system disease, hearing impairment-related disease, optic neuropathy, myopathy, cardiac dysfunction, liver dysfunction, kidney dysfunction, pancreatic dysfunction, gastrointestinal dysfunction, metabolic disease, reproductive disease, or skeletal lesion caused by the mitochondrial dysfunction;
optionally, the nervous system disease comprises at least one of neuronal development delay, Parkinson's disease, Alzheimer's disease, stroke, epilepsy, migraine, or amyotrophic lateral sclerosis;
optionally, the hearing impairment-related disease comprises at least one of deafness or sensorineural hearing loss;
optionally, the optic neuropathy comprises at least one of progressive external ophthalmoplegia, optic atrophy, or retinitis pigmentosa;
optionally, the myopathy comprises at least one of progressive muscle weakness and atrophy, sarcopenia, or exercise intolerance;
optionally, the cardiac dysfunction comprises at least one of cardiomyopathy or a myocardial conduction defect;
optionally, the liver dysfunction comprises at least one of liver failure, liver damage, fatty liver, or liver cirrhosis;
optionally, the liver dysfunction comprises at least one of Fanconi syndrome, renal tubular acidosis, glomerulosclerosis, renal failure, or adrenal insufficiency;
optionally, the pancreatic dysfunction comprises at least one of diabetes mellitus or pancreatitis;
optionally, the gastrointestinal dysfunction comprises at least one of intestinal pseudo-obstruction or gastrointestinal motility disorder;
optionally, the metabolic disease comprises at least one of senility, obesity, hyperglycemia, dyslipidemia, insulin resistance, or cardiovascular disease;
optionally, the reproductive disease comprises at least one of premature ovarian failure or male infertility; and
optionally, the skeletal disease comprises at least one of kyphoscoliosis, short stature, or bone marrow failure.
